# EUROPEAN PATENT APPLICATION

(11) **EP 4 257 677 A1**
(43) Date of publication of application: **11.10.2023**
(21) Application number: 21899849.0
(22) Date of filing: 16.11.2021
(51) Int. Cl.: C12N 5/10, C12N 15/867, C12N 7/01, C12R 1/91

(54) **STABLE LENTIVIRUS PACKAGING CELL LINE AND PREPARATION METHOD THEREFOR**

(30) Priority: 04.12.2020 CN 202011403323
(71) Applicant: Beijing Immunochina Pharmaceuticals Co., Ltd., Beijing 100195 (CN); Beijing Yimiaoyiliao Co., Ltd., Beijing 102629 (CN)
(72) Inventor: QI, Feifei, Beijing 102629 (CN); LU, Xin'an, Beijing 100195 (CN); HE, Ting, Beijing 100195 (CN); DENG, Cuiyun, Beijing 100195 (CN)
(74) Representative: BOVARD AG
(86) International application number: PCT/CN2021/130967
(87) International publication number: WO 2022/116815

(57) **Abstract**

Provided are a stable lentivirus packaging cell line and a preparation method therefor. The stable lentivirus packaging cell line contains a packaging plasmid group consisting of a pPuro.coTetR plasmid, a pVSVG plasmid, and a pGagPol-RRE-NES-cINT plasmid, wherein the pPuro.coTetR plasmid contains a CoTetR gene, the pVSVG plasmid contains a VSVG gene, and the pGagPol-RRE-NES-cINT plasmid contains GagPol and Rev genes. The stable lentivirus packaging cell line is relatively stable in terms of passage, virus production, and a genetic gene copy number, and the lentivirus produced by using the stable lentivirus packaging cell line has a high titer and a low impurity content.

## Description

### TECHNICAL FIELD

This invention belongs to the fields of immunology and molecular biology, especially to the field of cell therapy. In particular, it relates to a stable lentivirus packaging cell line and a preparation method therefor.

### BACKGROUND

Lentivirus, a vector for gene therapy, comprises human immunodeficiency virus (HIV), equine infectious anaemia virus (EIAV), simian immunodeficiency virus (SIV), feline immunodeficiency virus (FIV), etc. Unlike other retroviral vectors, lentiviral vectors can infect both dividing and non-dividing cells. Lentivirus can infect various types of cells such as cardiomyocytes, tumor cells, neuron cells, stem cells, hepatocytes, and endothelial cells. It can accommodate larger foreign fragments and achieve stable expression of target genes while eliciting lower levels of immune responses, thus providing good gene therapy effectiveness. Accordingly, it has broader application prospects.

Lentiviruses are currently in most cases generated by transiently transfecting cells with packaging plasmids and expression plasmids. However, lentiviruses generated by the existing preparation processes have low titer, excessive impurity residues, and also unstable virus yields between batches. Therefore, there remains a need to construct a stable lentivirus packaging cell line which enables high-titer lentivirus production and a low content of impurities.

### SUMMARY

To this end, an object of the present invention is to provide a stable lentivirus packaging cell line and a preparation method therefor. The stable lentivirus packaging cell line is relatively stable in terms of passage, virus production, and copy numbers of genetic genes. Moreover, the lentivirus produced by using the stable lentivirus packaging cell line has a high titer and a low content of impurities.

To achieve the above object, one aspect of the present invention provides a stable lentivirus packaging cell line, which comprises a packaging plasmid set consisting of a pPuro.coTetR plasmid, a pVSVG plasmid and a pGagPol-RRE-NES-cINT plasmid.

The pPuro.coTetR plasmid comprises a CoTetR gene; the pVSVG plasmid comprises a VSVG gene; and the pGagPol-RRE-NES-cINT plasmid comprises GagPol and Rev genes.

In preferred embodiments of the present invention, the pPuro.coTetR plasmid further comprises a CMV promoter, a chimeric intron, an SV40 promoter, a puromycin resistance gene, and a polyadenylation signal.

The pVSVG plasmid further comprises a hybrid promoter consisting of a CMV promoter and two tetracycline operators, an SV40 promoter, a bleomycin resistance gene, and a polyadenylation signal.

The pGagPol-RRE-NES-cINT plasmid further comprises a hybrid promoter consisting of a CMV promoter and two tetracycline operators, a chimeric intron, an SV40 promoter, a hygromycin resistance gene, a cPPT/CTS element, a RRE element, and a polyadenylation signal.

In preferred embodiments of the present invention, the CoTetR gene has a sequence as shown in SEQ ID NO: 1; the VSVG gene has a sequence as shown in SEQ ID NO: 2; and the GagPol and Rev genes have a sequence as shown in SEQ ID NO: 3 and 4, respectively.

In preferred embodiments of the present invention, the pPuro.coTetR plasmid has a sequence as shown in SEQ ID NO: 5; the pVSVG plasmid has a sequence as shown in SEQ ID NO: 6; and the pGagPol-RRE-NES-cINT plasmid has a sequence as shown in SEQ ID NO: 7.

In preferred embodiments of the present invention, the cell line is one selected from the group consisting of HEK293, HEK293-T, HEK293-SF, TE671, HT1080 or HeLa cell lines.

Preferably, the cell line is a HEK293 cell line.

Based on the same inventive concept, another aspect of the present invention provides a method for preparing the above stable lentivirus packaging cell line, which comprises the steps of:
1) linearizing the pPuro.coTetR plasmid, pVSVG plasmid and pGagPol-RRE-NES-cINT plasmid by digestion with an enzyme, respectively;
2) co-transfecting cells with the linearized pPuro.coTetR plasmid and pVSVG plasmid, and adding a first antibiotic for screening to obtain first positive monoclonal cells; and
3) transfecting the first positive monoclonal cells with the linearized pGagPol-RRE-NES-cINT plasmid, and adding a second antibiotic for screening to obtain second positive monoclonal cells, i.e., stable lentivirus packaging cell lines.

In preferred embodiments of the present invention, in step 2), polyethyleneimine is further added during co-transfection of cells with the linearized pPuro.coTetR plasmid and pVSVG plasmid. The mass ratio of polyethyleneimine to the plasmids is polyethyleneimine: (pPuro.coTetR plasmid + pVSVG plasmid) = (1-8): 1;
preferably, the mass ratio of polyethyleneimine to the plasmids is: polyethyleneimine: (pPuro.coTetR plasmid + pVSVG plasmid) = (2-5): 1;
more preferably, the mass ratio of polyethyleneimine to the plasmids is: polyethyleneimine: (pPuro.coTetR plasmid + pVSVG plasmid) = 4:1; and/or,
in step 3), polyethyleneimine is further added during the transfection of the first positive monoclonal cells with the linearized pGagPol-RRE-NES-cINT plasmid; and the mass ratio of polyethyleneimine to the pGagPol-RRE-NES-cINT plasmid is (1-8): 1;
preferably, the mass ratio of polyethyleneimine to the pGagPol-RRE-NES-cINT plasmid is (2-5):1;
more preferably, the mass ratio of polyethyleneimine to the pGagPol-RRE-NES-cINT plasmid is 4:1.

In preferred embodiments of the present invention, in step 2), the pPuro.coTetR plasmid and the pVSVG plasmid are both used in an amount of (0.2-1.0) µg/10⁶ cells;
preferably, the pPuro.coTetR plasmid and the pVSVG plasmid are both used in an amount of (0.4-0.9) µg/10⁶ cells;
more preferably, the pPuro.coTetR plasmid and the pVSVG plasmid are both used in an amount of 0.8 µg/10⁶ cells; and/or,
in step 3), the pGagPol-RRE-NES-cINT plasmid is used in an amount of (0.2-1.0) µg/ 10⁶ first positive monoclonal cells;
preferably, the pGagPol-RRE-NES-cINT plasmid is used in an amount of (0.4-0.9) [µg/10⁶ first positive monoclonal cells;
more preferably, the pGagPol-RRE-NES-cINT plasmid is used in an amount of 0.8 [µg/10⁶ first positive monoclonal cells.

In preferred embodiments of the present invention, in step 2), the first antibiotic is a combination of puromycin and bleomycin; and puromycin and bleomycin are used in an amount of 1-5 µg/mL and 300-500 µg/mL, respectively; more preferably, puromycin and bleomycin are used in an amount of 2 µg/mL and 400 µg/mL, respectively; and/or,
in step 3), the second antibiotics are a combination of puromycin, bleomycin and hygromycin, wherein puromycin, bleomycin and hygromycin are used in an amount of 1-5 µg/mL, 300-500 µg/mL and 300-500 µg/mL, respectively; more preferably, puromycin, bleomycin and hygromycin are used in an amount of 2 µg/mL, 400 µg/mL and 400 µg/mL, respectively.

Based on the same inventive concept, another aspect of the present invention provides a method for producing lentivirus, wherein the above stable lentivirus packaging cell line is transfected with the target plasmids.

In preferred embodiments of the present invention, the method for producing lentivirus comprises:
transfecting the stable lentivirus packaging cell line with the target plasmids, and then adding an inducing agent to induce the stable lentivirus packaging cell line to produce lentiviruses.

In preferred embodiments of the present invention, polyethyleneimine is further added during the transfection of the stable lentivirus packaging cell line with the target plasmids; and the mass ratio of polyethyleneimine to the target plasmids is polyethyleneimine: the target plasmids = (1-8):1;
preferably, the mass ratio of polyethyleneimine to the target plasmids is polyethyleneimine: the target plasmids = (2-5): 1;
more preferably, the mass ratio of polyethyleneimine to the target plasmids is polyethyleneimine: the target plasmids = 4:1.

In preferred embodiments of the present invention, the target plasmid is used in an amount of (0.2-0.8) µg/10⁶ stable lentivirus packaging cell lines;
preferably, the target plasmid is used in an amount of (0.3-0.5) [µg/10⁶ stable lentivirus packaging cell lines;
more preferably, the target plasmid is used in an amount of 0.4 [µg/10⁶ stable lentivirus packaging cell lines.

In preferred embodiments of the present invention, the inducing agent is doxycycline; preferably, the time for adding the inducing agent is 20-30h after transfection; more preferably, the time for adding the inducing agent is 24h after transfection;
preferably, the inducing agent is added at a concentration of 1-5 µg/mL; more preferably, the inducing agent is added at a concentration of 2 µg/mL.

In preferred embodiments of the present invention, an enhancing agent is further added during the production of lentivirus to increase the lentiviral expression level;
the enhancing agent is sodium butyrate;
preferably, the enhancing agent is added at a concentration of 5-15mM; more preferably, the enhancing agent is added at a concentration of 10mM;
preferably, the time for adding the enhancing agent is 20-30 hours after transfection; more preferably, the time for adding the enhancing agent is 24 hours after transfection;
preferably, the time for changing the enhancing agent is 6-8 hours after addition of the enhancing agent; and/or,
the time for collecting the lentivirus is 45-50 hours; preferably, the time for collecting the lentivirus is 48 hours.

### BRIEF DESCRIPTION OF THE DRAWINGS

Fig. 1 shows plasmid maps of the packaging cell line; wherein Fig. 1a is a plasmid map of the pPuro.coTetR plasmid; Fig. 1b is a plasmid map of the pVSVG plasmid; and Fig. 1c is a plasmid map of the pGagPol-RRE-NES-cINT plasmid;
Fig. 2 shows agarose gel electrophoresis charts of the CoTetR, VSVG and GagPol genes of monoclonal cells Clone1, 5, 6, 7, 14, 17, 18, 19, 23, 24, 26, 27, 28, 31, 32 and 33 identified by semi-quantitative PCR;
Fig. 3 is a histogram showing validation of virus production by the lentivirus packaging cell line transfected with the target plasmids;
Fig. 4 is a histogram showing the optimized experiment results of transfection and induction of virus production for Clone31;
Fig. 5 is a histogram showing the results of the passage stability assay for Clone31;
Fig. 6 is a histogram showing the results of the virus production stability assay for Clone31;
Fig. 7 is a histogram showing the results of copy number stability assay for Clone31 cells; wherein Fig. 7a is a histogram showing the results of VSVG gene copy number stability assay for Clone31; Fig. 7b is a histogram showing the results of GagPol gene copy number stability assay for Clone31; and Fig. 7c is a histogram showing the results of Rev gene copy number stability assay for Clone31;
Fig. 8 is a comparison chart of viral titers between batches for Clone31 and 293 cells;
Fig. 9 is a comparison chart of viral titer VP (P24) for Clone31 and 293 cells;
Fig. 10 is a scaled-up comparison chart between Clone31 and 293 cells; wherein, Fig. 10a is a comparison chart of viability rates at 24h and 48h after transfection in E1000; Fig. 10b is a comparison chart of HCP residues after transfection in E1000; and Fig. 10c is a comparison chart of VP after transfection in E1000.

### DETAILED DESCRIPTION OF THE INVENTION

It should be noted that, unless otherwise defined, the technical terms or scientific terms used in one or more examples of the present description shall have the general meanings as understood by those skilled in the art to which the present disclosure pertains.

The experimental methods used in the following examples are conventional methods unless otherwise specified. The culture medium, reagent materials and kits used in the following examples are all commercially available products unless otherwise specified.

It should be noted that the concentrations of antibiotics (puromycin, bleomycin and hygromycin), inducing agent (doxycycline) and enhancing agent (sodium butyrate) as used herein all refer to their final concentrations in the culture medium. For example, the concentration of sodium butyrate to be added being 5-15 mM means that the amount of sodium butyrate to be added is such that the final concentration of sodium butyrate in the culture medium is 5-15 mM.

A lentiviral vector system is developed by using lentivirus as the backbone, and contains a packaging structure and a vector structure. A lentiviral packaging system generally consists of two parts: a lentiviral packaging plasmid and a lentiviral expression plasmid. The lentiviral packaging plasmid consists of sequences encoding viral proteins. The lentiviral expression plasmid contains long terminal repeats, packaging signals, and other genetic information required for packaging, transfection, and stable integration. The packaging plasmid and expression plasmid are used to co-transfect cells. The virus is packaged in the cells and secreted into the extracellular medium to produce viral particles that can be used to infect host cells.

In most cases, the existing lentiviruses are produced by transiently transfecting 293 or 293T cells cultured in cell factories with packaging plasmids and expression plasmids; wherein, the packaging plasmids contain long terminal repeats, packaging signals, and other genetic information required for packaging, transfection and stable integration; and the expression plasmid contains a target gene.

The large-scale production of lentiviruses by transient transfection of 293 cells is feasible and shows good prospects. However, transient transfection needs to be further optimized in the production process. The main bottleneck is that the transfection process requires a large amount of plasmid DNAs, thus involving expensive costs, difficulty in scale-up and unsustainable production of viruses. The viruses produced by transient transfection that contain excessive residual impurities (especially host cell proteins and DNA residues) incur more difficulties in the subsequent purification process, and also lead to unstable virus yields between batches, thus bringing about uncertainty in the arrangement of downstream processes. Therefore, it is necessary to construct a stable lentivirus packaging cell line which can stably integrate the vector elements required for packaging the virus into the genome of the host cell, and enable it to be genetically stable and convenient for large-scale production, and thus secure sustainable virus production.

An object of the present invention is to construct a stable lentivirus packaging cell line to realize the large-scale production of suspension cells (especially 293 cells), thereby resulting in high-titer lentiviruses with a low content of impurities.

To achieve the above object, one aspect of the present invention provides a stable lentivirus packaging cell line, which comprises a packaging plasmid set consisting of a pPuro.coTetR plasmid, a pVSVG plasmid and a pGagPol-RRE-NES-cINT plasmid.

The pPuro.coTetR plasmid comprises a CoTetR gene; the pVSVG plasmid comprises a VSVG gene; and the pGagPol-RRE-NES-cINT plasmid comprises GagPol and Rev genes.

As known in the prior art, the method of producing lentiviruses comprises transfecting cells (such as 293 or 293T cells) with a packaging plasmid and an expression plasmid simultaneously. It has the main disadvantages of low-titer lentivirus production, a high content of impurities, and also unstable viral yields between batches. The method of this invention comprises initially transfecting a cell line with packaging plasmids to obtain a stable lentivirus packaging cell line, i.e., transfecting a cell line with the pPuro.coTetR plasmid, pVSVG plasmid and the pGagPol-RRE-NES-cINT plasmid to obtain a stable lentivirus packaging cell line, and then transfecting the stable lentivirus packaging cell line with the target plasmid to produce high-titer lentiviruses with a low content of impurities.

The stable lentivirus packaging cell lines as constructed according to the present invention were studied for stability, including passage stability, virus production stability and copy number stability. The experimental results indicate that the stable lentivirus packaging cell lines as constructed herein can be stably passed down from generation to generation in the absence of selective pressure (without adding an antibiotic), and have a viability rate of 97% or more, suggesting that the constructed cells can be stably passed down over 30 generations or more in the absence of selective pressure; the cells passed down without antibiotics can be inherited normally, have the ability to produce lentiviruses, and contain stable copy numbers of the GagPol gene, Rev gene and VSVG gene. In other words, the stable lentivirus packaging cell lines as constructed herein can remain stable in terms of passage, virus production and copy numbers of genetic genes.

The pPuro.coTetR plasmid, pVSVG plasmid and pGagPol-RRE-NES-cINT plasmid of the present invention are all constructed by using a commercialized plasmid backbone as the basis and then inserting a target gene into it. For example, the pPuro.coTetR plasmid is constructed by using the pIRESpuro3 plasmid as the basis and then inserting a CoTetR gene, wherein the CoTetR gene is a codon-optimized TetR (tetracycline repressor) gene and has a sequence as shown in SEQ ID NO: 1. The pVSVG plasmid is constructed by using pcDNA4 plasmid as the basis and then inserting a VSVG (vesicular stomatitis virus glycoprotein) gene, wherein the VSVG gene has a sequence as shown in SEQ ID NO: 2. The pGagPol-RRE-NES-cINT plasmid is constructed by using pcDNA5 plasmid as the basis and then inserting a GagPol (wherein, the Gag gene encodes a virus capsid protein, and the Pol gene encodes a virus reverse transcriptase and gene integrase) and a Rev gene (which encodes a regulator of viral particle protein expression), wherein the GagPol gene and Rev gene have a sequence as shown in SEQ ID NO: 3 and 4, respectively.

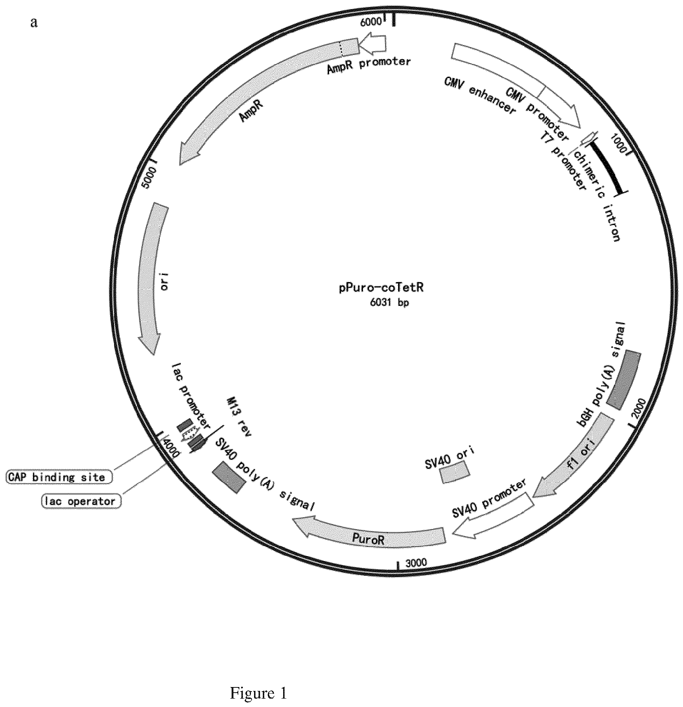

As shown in Fig. 1a, the pPuro.coTetR plasmid further comprises a human cytomegalovirus (CMV) promoter, a chimeric intron (which increases protein expression levels), a Simian virus 40 (SV40) promoter, puromycin resistance gene (Puro^{R}) and a polyadenylation signal. The SV40 promoter is required for expression of the puromycin resistance gene. The pPuro.coTetR plasmid comprises a puromycin resistance gene. After having been screened with puromycin, almost all viable cells will stably express the CoTetR gene, and thus there is no need to screen a large number of cells for functional clones. When the pPuro.coTetR plasmid is used, the antibiotic will exert selective pressure across the entire expression cassette. Thus, high doses of antibiotics will select cells expressing high levels of the CoTetR gene. This selective pressure also ensures that the expression of the CoTetR gene will remain stable. The pPuro.coTetR plasmid has a sequence as shown in SEQ ID NO:5.

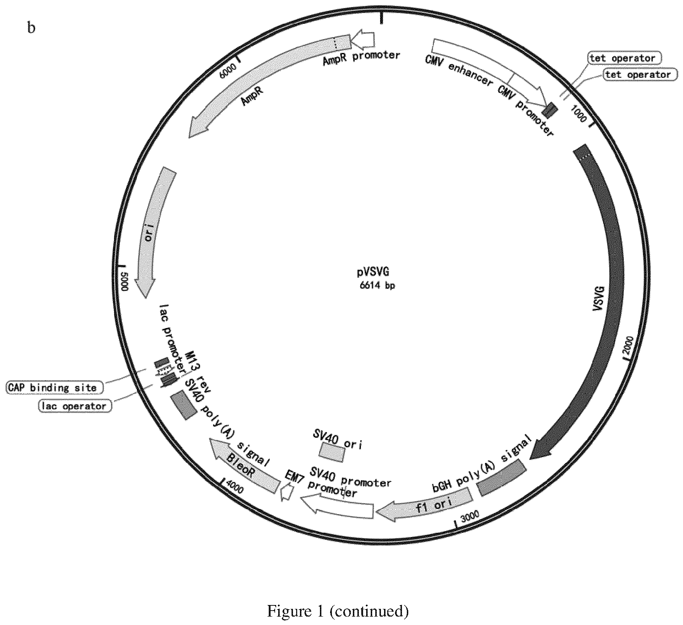

As shown in Fig. 1b, the pVSVG plasmid further comprises a hybrid promoter consisting of a human cytomegalovirus (CMV) promoter and two tetracycline operators (TetO₂), a Simian virus 40 (SV40) promoter, a bleomycin resistance gene (Zeo^{R}), and a polyadenylation signal. The SV40 promoter is required for expression of the bleomycin resistance gene. The pVSVG plasmid has a sequence as shown in SEQ ID NO:6.

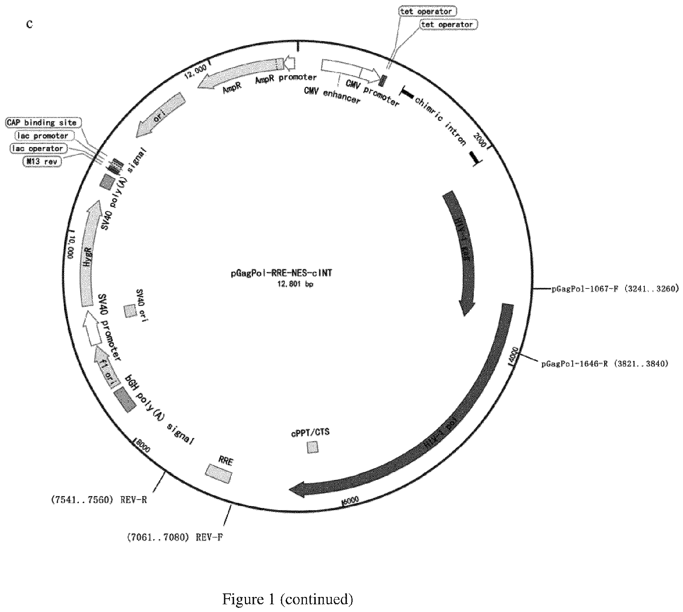

As shown in Fig. 1c, the pGagPol-RRE-NES-cINT plasmid further comprises a hybrid promoter consisting of a human cytomegalovirus (CMV) promoter and two tetracycline operators (TetO₂), a chimeric intron, an Simian virus 40 (SV40) promoter, a hygromycin resistance gene (Hygro^{R}), a cPPT/CTS element, a RRE element, and a polyadenylation signal. The SV40 promoter is required for expression of the hygromycin resistance gene. The pGagPol-RRE-NES-cINT plasmid has a sequence as shown in SEQ ID NO: 7.

The pGagPol-RRE-NES-cINT plasmid comprises a human immunodeficiency virus (HIV) rev response element (RRE), which is a retroviral export element that regulates the transport of RNA transcripts from the nucleus to the cytoplasm.

The pGagPol-RRE-NES-cINT plasmid comprises cis-acting elements - a central polypurine region (cPPT) and a central termination sequence (CTS). The cPPT/CTS sequence can be the cPPT/CTS of HIV1, which can improve vector integration and transduction efficiency.

In the present invention, the hybrid promoter consisting of a human cytomegalovirus (CMV) promoter and two tetracycline operators (TetO₂) means inserting two TetO₂ operator sequences into the CMV promoter to confer the function of regulating the promoter to tetracycline. The TetO₂ sequence consists of 2 copies of a 19-nucleotide sequence (5'-TCCCTATCAGTGATAGAGA-3').

Based on the same inventive concept, another aspect of the present invention provides a method for preparing the above stable lentivirus packaging cell line, which comprises the steps of:
1) linearizing the pPuro.coTetR plasmid, pVSVG plasmid and pGagPol-RRE-NES-cINT plasmid by digestion with an enzyme, respectively;
2) co-transfecting cells with the linearized pPuro.coTetR plasmid and pVSVG plasmid, and adding first antibiotics for screening to obtain first positive monoclonal cells; and
3) transfecting the first positive monoclonal cells with the linearized pGagPol-RRE-NES-cINT plasmid, and adding second antibiotics for screening to obtain second positive monoclonal cells, i.e., stable lentivirus packaging cell lines.

Since the pPuro.coTetR plasmid and the pVSVG plasmid of the present invention comprise a puromycin resistance gene and a bleomycin resistance gene, respectively, puromycin and bleomycin are added during the process of screening for the first positive clone cells to perform resistance screening. Meanwhile, since the pGagPol-RRE-NES-cINT plasmid comprises a hygromycin resistance gene, puromycin, bleomycin and hygromycin are added during the process of screening for the second positive clone cells to perform resistance screening. In addition, polyethyleneimine (PEI) needs to be added during the two transfection processes to improve the transfection efficiency, and the stable lentivirus packaging cell line of the present invention can be obtained by controlling the amounts of PEI and each plasmid.

Based on the same inventive concept, another aspect of the present invention provides a method of producing a lentivirus, wherein the above stable lentivirus packaging cell line is transfected with a target plasmid (an expression plasmid comprising a target gene).

In preferred embodiments of the present invention, the method of producing a lentivirus comprises:
transfecting the stable lentivirus packaging cell line with the target plasmid, and then adding an inducing agent to induce the stable lentivirus packaging cell line to produce lentiviruses.

The stable lentivirus packaging cell line of the present invention is an induction system. During the production of lentiviruses, it is necessary to add doxycycline (dox) to induce virus production, and it was found that adding sodium butyrate during the virus production process can increase the yield of lentiviruses. The present invention also optimizes the conditions of transfecting the stable lentivirus packaging cell line with the target plasmids and inducing virus production. The optimal conditions are: the amount of the target plasmid being 0.4 µg/10⁶ cells, the PEI amount: the target plasmids amount=4:1, the concentration of sodium butyrate being 10mM, the time for adding sodium butyrate being 24h after transfection, the time for change of sodium butyrate being 6-8 hours after the addition of sodium butyrate, the time for adding dox being 24 hours, the concentration of dox being 2 µg/mL, and the time for collecting lentiviruses being 48h. There is no need to add doxycycline (dox) and sodium butyrate in the method for producing lentiviruses by transient transfection with four plasmids in the prior art.

In the present invention, the target plasmid is not limited. Various target plasmids can be used for transfection as required. Preferably, the target plasmid is a CAR expression plasmid.

Chimeric antigen receptor (CAR) T-cell immunotherapy is an adoptive immune-cell therapy, in which an scFv fragment that can recognize tumor-specific antigens is genetically engineered and fused with a transmembrane segment consisting of molecules involved in the activation of T cells such as CD28, 4-1BB, and then the gene segment is transfected into the T cells extracted from the peripheral blood of the patient through lentivirus or retrovirus gene transduction; after the genetically modified T cells are re-infused into the patient, the expressed CAR receptors are used to bind to molecules on the surface of tumor cells, thereby generating internal signals to activate T cells to quickly kill tumor cells. The CAR-T cells have integrated the recognition specificity of antibodies and the killing effect and memory function of T cells. Thus, they can recognize tumor antigens without being restricted by MHC, and are capable of recognizing a broader range of targets than natural T cells. Based on actual needs, the above-mentioned stable lentivirus packaging cell line can be transfected with CAR expression plasmids for the treatment of relapsed/refractory CD19-positive non-Hodgkin's lymphoma. For the design and vector construction of CAR, reference may be made to the published text of the Chinese invention patent application 201510324558.X, especially its Examples 1 and 2, to design a chimeric antigen receptor (CAR) comprising a signal peptide, an extracellular binding region, an optional hinge region, a transmembrane region and an intracellular signal region (which are sequentially linked) and construct a vector (such as IM19 plasmid).

In the present invention, transfection of the above-mentioned stable lentivirus packaging cell line with CAR expression plasmids is compared with the four-plasmid transient transfection (i.e., 293 cells are transfected simultaneously with pPuro.coTetR plasmid, pVSVG plasmid, pGagPol-RRE-NES-cINT plasmid, and CAR expression plasmid) known in the prior art, in terms of their cell viability, viral titer VP (P24), host cell protein (HCP) residues and viral titer stability between batches. The experimental results show that the stable lentivirus packaging cell line as constructed herein has high viral titer VP content and stable viral yields between batches, which are advantageous for the downstream purification process.

Furthermore, the above-mentioned stable lentivirus packaging cell line transfected with the CAR expression plasmid is compared in scaled-up processes with the 293 cells transiently transfected with the four plasmids known in the prior art, in terms of their cell viability, viral titer VP, and host cell protein (HCP) residues. The experimental results show that the stable lentivirus packaging cell line has high cell viability after transfection, low host cell protein (HCP) residue in the viruses produced, and high viral titer VP, which can be used in large-scale virus production.

The VP in the viral titer VP (P24) refers to a method of measuring the viral titer - the viral particle number (VP) method - which uses OD260 to detect the number of viral particles. P24 refers to the virus capsid protein-the P24 protein. The viral titer is detected by measuring the P24 protein.

As is clear from the above disclosure, the present invention provides a stable lentivirus packaging cell line and a preparation method therefor. The stable lentivirus packaging cell line is relatively stable in terms of passage, virus production, and copy numbers of genetic genes; and the stable lentivirus packaging cell line is suitable for use in large-scale virus production. Compared with the 293 cells transiently transfected with the four plasmids, the stable lentivirus packaging cell line has high cell viability, high viral titer VP content, less host cell protein residues and stable virus yields between batches, which are advantageous for the downstream purification process.

The technical solutions provided by the present invention will be further described below in conjunction with specific examples. The following examples are only used to illustrate the present invention, and do not to any extent limit the protection scope thereof.

### Example 1 Construction of pPuro.coTetR plasmid, pVSVG plasmid and pGagPol-RRE-NES-cINT plasmid

The stable lentivirus packaging cell line as constructed in this example consists of three plasmids: pPuro.coTetR (comprising a CoTetR gene), pVSVG (comprising a VSVG gene), and pGagPol-RRE-NES-cINT (comprising GagPol and Rev genes), which have plasmid maps as shown in Fig.1a, Fig.1b and Fig.1c, respectively.

### Construction of pPuro-coTetRn vector:

An INT fragment (230 bp) was obtained by using TetR-WF-1F/TetR-1R-linker as primer and pIRESpuro3 (TaKaRa, cat.no.: 631619) as template; a coTetR fragment (624 bp) was obtained by using TetR-2F-linker/TetR-2R-linker as primer and coTetR as template (which has a sequence as shown in SEQ ID NO: 1); and a PA+F1ori+SV40promoter fragment (1042 bp) was obtained by using TetR-3F-linker/TetR-WF-3R as primer and pcDNA4 (Thermo, cat.no.: V102020) as template. Then, by bridging PCR, the primer TetR-WF-1F/TetR-2R-linker was used to obtain the fragment TNT+coTetR, and the primer TetR-WF-1F/TetR-WF-3R was used to obtain the full-length fragment TNT+coTetR+PA+F1ori+SV40 promoter.

The full-length fragment prepared above was inserted into the pIRESpuro3 vector at the HindIII site by seamless cloning method, and transformed into competent stbl3 (full gold, cat.no.: CD521). After sequencing for confirming correct insertion, the plasmid was extracted with the EndoFree Plasmid Midi Kit (CWBio, cat.no.: cw2105s). The constructed target plasmid was used in the subsequent experiments of constructing the packaging cell lines.

| Primer Name | Sequences of primers |
|---|---|
| TetR-WF-1F | 5'-ACTCACTATAGGGAGACCCA GTGAGTACTCCCTCTCAAAA-3' |
| TetR-1R-linker | 5’-TTGGACTTGTCCAGCCTAGACATCTGTGGAGAGAAAGGCAAAGT GGAT-3’ |
| TetR-2F-linker | 5’-ATCCACTTTGCCTTTCTCTCCACAGATGTCTAGGCTGGACAAGTC CAA -3’ |
| TetR-2R-linker | 5'-ATGGCTGGCAACTAGAAGGCACAGTCAGCTGCCGCTTTCGCACT TGA -3' |
| TetR-3F-linker | 5’-TCAAGTGCGAAAGCGGCAGCTGACTGTGCCTTCTAGTTGCCAGC CAT-3’ |
| TetR-WF-3R | 5'-CTTGTGGGTTGTGGCAAGCT TTTGCAAAAGCCTAGGCCTC -3' |

### Construction of pVSVG vectors:

A VSVG fragment (1536bp) was obtained by using VSVG-WF-F/VSVG-WF-R as primer and VS-SC (which has a sequence as shown in SEQ ID NO: 8) as template. The fragment prepared above was inserted into the pcDNA4 vector at XhoI site by seamless cloning method, and transformed into competent stbl3. After sequencing for confirming correct insertion, the plasmid was extracted with the EndoFree Plasmid Midi Kit (available from CWBio). The constructed target plasmid was used in the subsequent experiments of constructing the packaging cell lines.

| Primer Name | Sequences of primers |
|---|---|
| VSVG-WF-F | 5'-ATATCCAGCACAGTGGCGGCCGCATGAAGTGCCTTTTGTACTT-3' |
| VSVG-WF-R | 5'-AACGGGCCCTCTAGACTCGATTACTTTCCAAGTCGGTTCA-3' |

### Construction of pGagPol-RRE-NES-cINT vectors:

A GagPol fragment (4307 bp) was obtained by using pGagPol-XhoI-WF/pGagPol-XhoI-WR as primer and MDK-SC (which has a sequence as shown in SEQ ID NO: 9) as template. The fragment prepared above was inserted into the pcDNA5 vector (Thermo, Cat. No.: V103320) at XhoI site by seamless cloning method, and transformed into competent stbl3. After sequencing for confirming correct insertion, the plasmid was extracted with the EndoFree Plasmid Midi Kit available from CWBio). The constructed target plasmid pGagPol was used in the subsequent A RRE-NES fragment (1705 bp) was obtained by using pGagPol-REV-Xba I-WF/pGagPol-REV-Xba I-WR as primer and AX (also known as psPAX2 vector, which has a sequence as shown in SEQ ID NO: 10) as template. The fragment prepared above was inserted into the pGagPol vector at XbaI site by seamless cloning method, and transformed into competent stbl3. After sequencing for confirming correct insertion, the plasmid was extracted with the EndoFree Plasmid Midi Kit (CWBio). The constructed target plasmid pGagPol-RRE-NES was used in the subsequent vector construction.

A cINT fragment (1122 bp) was obtained by using pGagPol-RN-cINT-Not I-QF/pGagPol-INT-Not I-WR as primer and AX as template. The fragment prepared above was inserted into the pGagPol-RRE-NES vector at Not I site by seamless cloning method, and transformed into competent stbl3. After sequencing for confirming correct insertion, the plasmid was extracted with the EndoFree Plasmid Midi Kit (available from CWBio). The constructed target plasmid pGagPol-RRE-NES-cINT was used in the subsequent vector construction.

| Primer Name | Sequences of primers |
|---|---|
| pGagPol-Xho I-WF | 5’-TCCAGCACAGTGGCGGCCGCATGGGTGCGAGAGCGTCAG T-3’ |
| pGagPol-Xho I-WR | 5’-AACGGGCCCTCTAGACTCGATTAATCCTCATCCTGTCTAC -3’ |
| pGagPol-REV-Xba I-WF | 5'-AGGATGAGGATTAATCGAGTCACATGGAATTCTGCAACA A -3' |
| pGagPol-REV-Xba I-WR | 5’-GGTTTAAACGGGCCCTCTAGAGCCAGAAGTCAGATGCTC A-3’ |
| pGagPol-RN-cINT--Not I-QF | 5’-CAGATATCCAGCACAGTGGCGGAGTCGCTGCGCGCTGCC T -3’ |
| pGagPol-INT-Not I-WR | 5' - GCTCTCGCACCCATGCGGCCCTCTCACCAGTCGCCGCC-3' |

### Example 2 Preparation of stable lentivirus packaging cell lines

The three plasmids as constructed in Example 1 were linearized by digestion with Fsp I (Thermo, cat.no.: FD1224), and then dephosphorylated to be used for transfecting 293 cells.

Linearized pPuro.coTetR and pVSVG plasmids were used to co-transfect 293 suspension cells with PEI (polyethyleneimine) (Polysciences, cat.no.: 23966). The pPuro.coTetR and pVSVG plasmids were both used for transfection in an amount of 0.8 [µg/10⁶ cells; the PEI amount: (the pPuro.coTetR plasmid amount +pVSVG plasmid amount) = 4:1. Pressurized selection was performed by using two antibiotics: 2 µg/mL Puro (puromycin) (InVivogen, cat.no.: ant-pr-1) and 400 µg/mL Zeo (bleomycin) (Invitrogen, cat.no.: R25001) for 2 weeks to obtain drug-resistant positive stably-transfected cell pools, i.e., polyclonal PV cells. The polyclonal PV cells were plated on a 96-well plate (Thermo, cat.no.: 167008) by limited dilution, at a density of 200 µL per well. Then the cells were observed under the microscope. The wells with single cells only were selected and labeled, and cultured in an incubator for about 20-30 days.

The monoclonal cells survived in the 96-well plate were amplified and cultured in 48-well plate, 24-well plate, 12-well plate, 6-well plate and shake flask E125, respectively. The PV monoclonal cells were taken to extract RNA. After reverse transcription into cDNA, semi-quantitative PCR was performed to identify the presence of the target genes CoTetR and VSVG. β-actin was used as an internal reference gene.

The PV monoclonal cells identified as correct were transfected with the linearized plasmid pGagPol-RRE-NES-cINT using PEI (polyethyleneimine). The pGagPol-RRE-NES-cINT plasmid was used for transfection in an amount of 0.8 [µg/10⁶ first positive monoclonal cells; the PEI amount: the pGagPol-RRE-NES-cINT plasmid amount = 4:1. Pressurized selection was performed by using three antibiotics: 2 µg/mL Puro (puromycin), 400 µg/mL Zeo (bleomycin), 400 µg/mL HYG (hygromycin) (Invitrogen, cat.no.: 10687010) for 2 weeks to obtain drug-resistant positive stably-transfected cell pools, i.e., polyclonal PV cells. The polyclonal PV cells were plated on a 96-well plate (Thermo, cat.no.: 167008) by limited dilution, at a density of 200 µL per well. Then the cells were observed under the microscope. The wells with single cells only were selected and labeled, and cultured in an incubator for about 20-30 days. The monoclonal cells survived in the 96-well plate were numbered as Clone1, Clone5, Clone6, Clone7, Clone14, Clone17, Clone18, Clone19, Clone23, Clone24, Clone26, Clone27, Clone28, Clone31, Clone32 and Clone33.

### Example 3 Identification of stable packaging cell lines by semi-quantitative PCR

The monoclonal cells survived in the 96-well plate were amplified and cultured in 48-well plate, 24-well plate, 12-well plate, 6-well plate and shake flask E125, respectively. The PVGR monoclonal cells were taken to extract RNA. After reverse transcription into cDNA, semi-quantitative PCR was performed to identify the presence of the target genes CoTetR, VSVG and GagPol. β-actin was used as an internal reference gene. Specific steps are as follows:
One day before the experiment, PVGR monoclonal cells were plated in 12-well plate at 3×10⁵ cells/mL per well, and cultured in media containing 1 mL of SMM293 TI (Sino Biological, cat.no.: M293TI) + 0.1% F68 (Gibco, cat.no.: 24040-032). 2 µg/mL dox (doxycycline) (Sigma, cat.no.: D9891) was added at day 2, and the cells were collected at day 3 for RNA extraction. The RNAs were extracted with TRIzol^{™} reagent (InvitrogenTM, cat.no.: 15596026) by the method according to the manufacturer's instructions.

Each of 2 µg extracted RNA was reverse-transcribed into cDNA using a first-strand cDNA synthesis kit (Thermo ScientificTM, Cat. No.: K1612) according to the manufacturer's instructions.

Each of 2 µg reverse transcribed cDNA was used for amplifying the target genes CoTetR, VSVG and GagPol by PCR, respectively. β-actin was used as an internal reference gene, and agarose gel electrophoresis was performed. The system for amplification of target genes by PCR includes: 10 µL 2×phanta Max master mix (Nanjing Vazyme, cat. no.: P515-02), 1 µL forward primer, 1 µL reverse primer, 7 µL ddH₂O, and 1 µL cDNA template. The PCR reaction program is: 95°C for 10 min, 30 cycles of 95°C for 30 s, 56°C for 30 s, 72°C for 30 s, 72°C for 10 min. Primers for semi-quantitative PCR are as follows:
CoTetR forward primer:
   5'-atccactttgcctttctctccacagatgtctaggctggacaagtccaa-3'
CoTetR reverse primer:
   5'-atggctggcaactagaaggcacagtcagctgccgctttcgcacttga-3'
VSVG forward primer: 5'-ccgctcgagatgaagtgccttttgtactt-3'
VSVG reverse primer: 5'-tgcattttccgttgatgaac-3'
GagPol forward primer: 5'-ccggccataaagcaagagtt-3'
GagPol reverse primer: 5'-ccgcagatttctatgagtat-3'
β-actin forward primer: 5'-CTCCATCCTGGCCTCGCTGT-3'
β-actin reverse primer: 5'-GCTGTCACCTTCACCGTTCC-3'

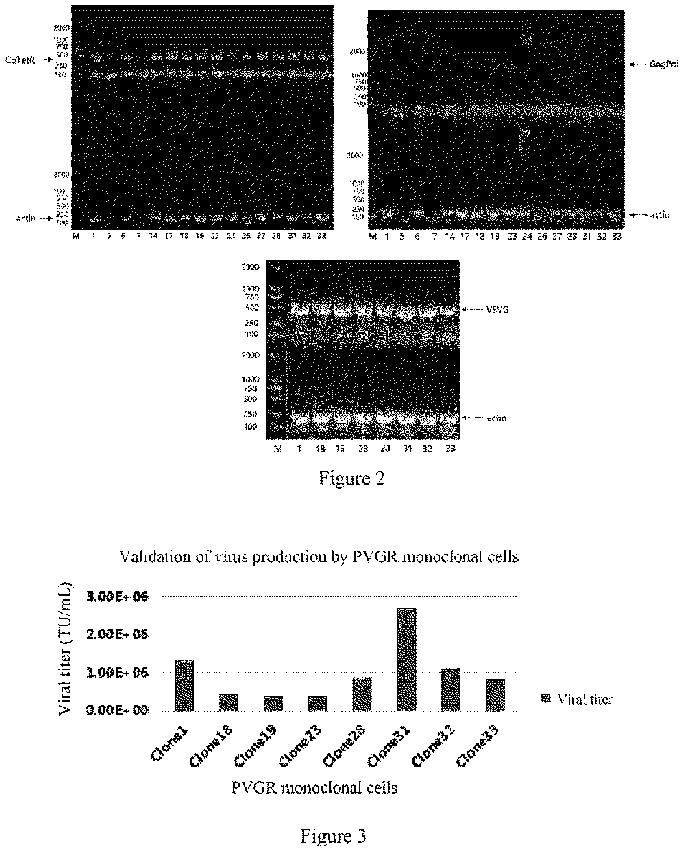

The experimental results are shown in Fig. 2. As shown in Fig. 2, the Clone1, Clone18, Clone19, Clone23, Clone28, Clone31, Clone32 and Clone33 all comprise target fragments CoTetR, VSVG and GagPol.

### Example 4 Construction of target plasmid (CAR expression plasmid)

### 4.1 Preparing nucleotide sequence of CAR molecule

The CAR molecule of this example comprises a signal peptide, an extracellular binding region, an optional hinge region, a transmembrane region, and an intracellular signal region. The nucleotide sequence of the extracellular binding region was obtained by humanized engineering based on the nucleotide sequence of the antigen binding region of the anti-CD19 chimeric antigen receptor (named here as anti-CD19scFv-S0, referred to as scFv-S0, which is derived from mice, see J Immunother. 2009 September; 32(7):689-702.). Steps for preparing the nucleotide sequence of the CAR molecule are specifically described.

First, primers are designed. The sequences for the primers used in this example are listed below:
1-1: 5'-ATGCTTCTCCTGGTGACAAG-3'
1-2: 5'-TGGGATCAGGAGGAATGCTG-3'
2-1: 5'-TACATCTGGGCGCCCTTGGCCGG-3'
2-2: 5' -GGAGCGATAGGCTGCGAAGTCGCG-3'
3-1: 5'-AGAGTGAAGTTCAGCAGGAGCG-3'
3-2: 5'-TTAGCGAGGGGGCAGGGCCT-3'
4-1: 5'-ATGCTTCTCCTGGTGACAAGCC-3'
4-2: 5'-TGAGGAGACGGTGACTGAGGTTCCTTGG-3'
5-1: 5'-GCGGCCGCAATTGAAGTTATGTA-3'
5-2: 5'-TTAGCGAGGGGGCAGGGCCTGC-3'
6-1: 5'-CTAGACTAGTATGCTTCTCCTGGTGACAAGCC-3'
6-2: 5'-CGACGCGTTTAGCGAGGGGGCAGGGCCTGC-3'

Using human cDNA library as template, 1-1 and 1-2, 2-1 and 2-2, 3-1 and 3-2 as primers, respectively, corresponding CAR molecules were cloned by PCR, which are GMCSF, CD28-TM+CD28-signal (the two parts are connected) and CD3ζ-signal. A GMCSF+scFv fragment was obtained by using bridging primers 4-1 and 4-2; a CD28-TM+CD28-signal+CD3ζ-signal fragment was obtained by using bridging primers 5-1 and 5-2; and then the nucleotide sequence of the complete CAR molecule was obtained by using bridging primers 6-1 and 6-2. The digestion sites were SpeI and MluI.

### 4.2 Construction of viral vectors comprising nucleic acid sequences of CAR molecules

The nucleotide sequence of the CAR molecule as prepared above was double digested with SpeI (Fermentas) and MluI (Fermentas), ligated with T4 ligase (Fermentas), inserted into the lentiviral IM19 vector at SpeI-MluI site, and transformed into competent *E*. *coli* (DH5a). After sequencing for confirming correct insertion, the plasmids were extracted with the plasmid purification kit (from OriGene Technologies, Inc.) and purified. The constructed target plasmids (CAR expression plasmids) were used for the subsequent virus production experiments.

### Example 5 Validation of virus production by stable packaging cell lines

In this example, the PVGR monoclonal cells identified as correct were validated for virus production; that is, the PVGR monoclonal cells of Example 3 were transiently transfected with the target plasmids as constructed in Example 4 for the validation of virus production. Specific steps are as follows:
One day before the experiment, PVGR monoclonal cells were plated in 12-well plate at a density of 8×10⁵ cells/mL per well, and cultured in media containing 1 mL of SMM293 TI + 0.1% F68. The target plasmids were used for transfection at day 2. At 24 hours post-transfection, an inducing agent dox (doxycycline) was added at a final concentration of 2 µg/mL. At 48 hours post-transfection, the viruses were collected, and centrifuged at 3000rpm under 4°C for 20min. Jurkat cells were infected with the viruses, and analyzed by flow cytometry after 48h to calculate the viral titer (TU/mL). The experimental results showed that Clone1, Clone18, Clone19, Clone23, Clone28, Clone31, Clone32 and Clone33 all had the ability to produce viruses, and Clone31 exhibited the highest viral titer of 2.68E+06 (i.e., 2.68× 10⁶) TU/mL (see Fig. 3). Clone31 was selected as the test cell in the subsequent experiments.

### Example 6 Experiments of optimizing the conditions for transfection of the stable packaging cell line with the target plasmids and for induction of virus production

The monoclonal Clone31 cells with relatively high viral titer were selected for further study. Clone31 cells were inoculated into shake flasks E125 (Corning, cat. no.: 431145) at a density of 8×10⁵ cells/mL in an amount of 10 mL, respectively, and cultured in media containing 10 mL SMM293 TI. Design of experiments (DOE) was used. First, a screening design experiment was used. The factors include: the amount of plasmid (target plasmid), amount of PEI (polyethyleneimine), concentration of sodium butyrate, time for adding sodium butyrate, time for change of sodium butyrate, time for adding dox (doxycycline), concentration of the added dox (doxycycline), and time for collecting the lentivirus. The effects of curvature and factors were determined. Second, a central composite design was used to determine the optimal experimental scheme. The viruses were collected 48 hours post-transfection, and centrifuged at 3000 rpm under 4°C for 20 minutes to determine viral titer TU.

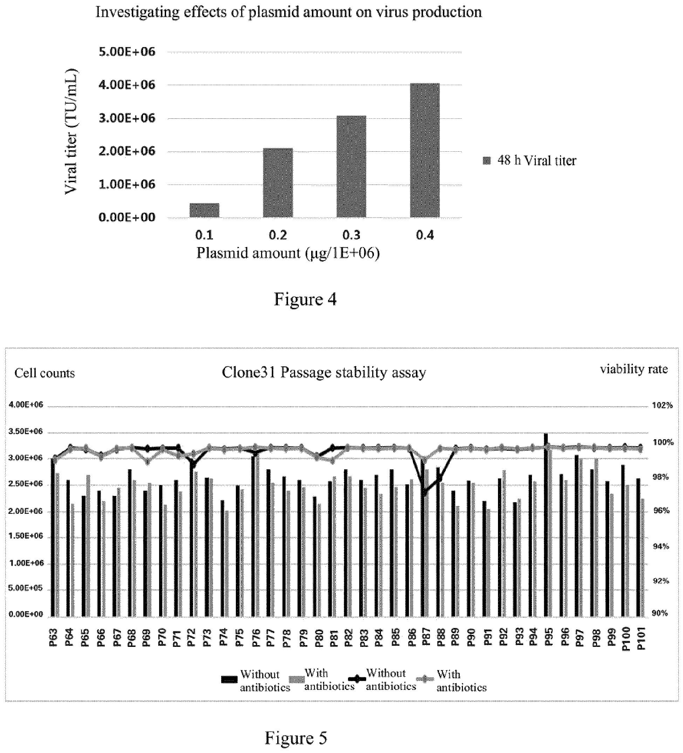

Finally, it can be concluded that the optimal experimental scheme is: the amount of plasmid (target plasmid) being 0.4 [µg/10⁶ cells, the PEI (polyethyleneimine) amount: plasmid (target plasmid) amount = 4:1, the concentration of sodium butyrate (Sigma, cat. no.: B5887) being 10 mM, the time for adding sodium butyrate being 24 hours post-transfection, the time for change of sodium butyrate being 6-8 hours after adding sodium butyrate, and the time for adding dox (doxycycline) being 24 hours, the concentration of the added dox (doxycycline) being 2 µg/mL, and the time for collecting the viruses being 48 h. Jurkat cells were infected with the collected viruses. After 48 hours, the cells were analysed by flow cytometry to calculate viral titer. Clone31 has a final optimized viral titer of 4E+06 (i.e., 4×10⁶) TU/mL in shake flask E125 (see Fig. 4).

### Example 7 Validation for passage, virus production, copy number stability of stable packaging cell lines

The stable lentivirus packaging cell line Clone31 was further used in stability studies, including passage stability, virus production stability and copy number stability.

### 7.1 Passage stability

Clone31 monoclonal cells were passaged in E125 shake flasks with and without antibiotics, and cultured at a volume of 10 mL each time. The cell count and viability rate were calculated, respectively. In particular, Clone31 cells were inoculated into E125 shake flasks (Corning, Cat: 431145) at a density of 8×10⁵ cells/mL and an volume of 10 mL, respectively. 10 mL of SMM293 TI media (without antibiotics), and 10 mL of SMM293 TI+0.6 µg/mL Puro+50 µg/mL Zeo+50µg/mL Hyg were used, respectively. The cells were passaged once every two days. The cell count and viability rate were calculated, respectively. The passage stability was observed.

Experiments have proved that when no antibiotics were added (in the absence of selective pressure), the cell counts of the Clone31 monoclonal cells were not considerably different from those with the addition of antibiotics; and that when no antibiotics were added (in the absence of selective pressure), Clone31 monoclonal cells can be stably passaged and have a viability rate of up to 97% or more, indicating that the cells can be stably passaged for 30 generations or more in the absence of selective pressure (see Fig. 5).

### 7.2 Virus yield stability

Virus yield stability of Clone31 monoclonal cells with and without antibiotics was investigated. With or without the addition of antibiotics, Clone31 was transfected with the target plasmids (as constructed in Example 4) every few generations, and the viral titer was measured. In particular, one day before the experiment, Clone31 cells were inoculated into E125 shake flasks (Corning, cat. no.: 431145), at a density of 1.4×10⁶ cells/mL and a volume of 10 mL respectively. 10 mL SMM293 TI medium (without antibiotics), and 10 mL SMM293 TI+0.6 µg/mL Puro (puromycin)+50 µg/mL Zeo (bleomycin)+50 µg/mL Hyg (hygromycin) were used, respectively. At day 2, the cells were transfected with the target plasmids (as constructed in Example 4). Transfection was performed every few generations to validate virus production.

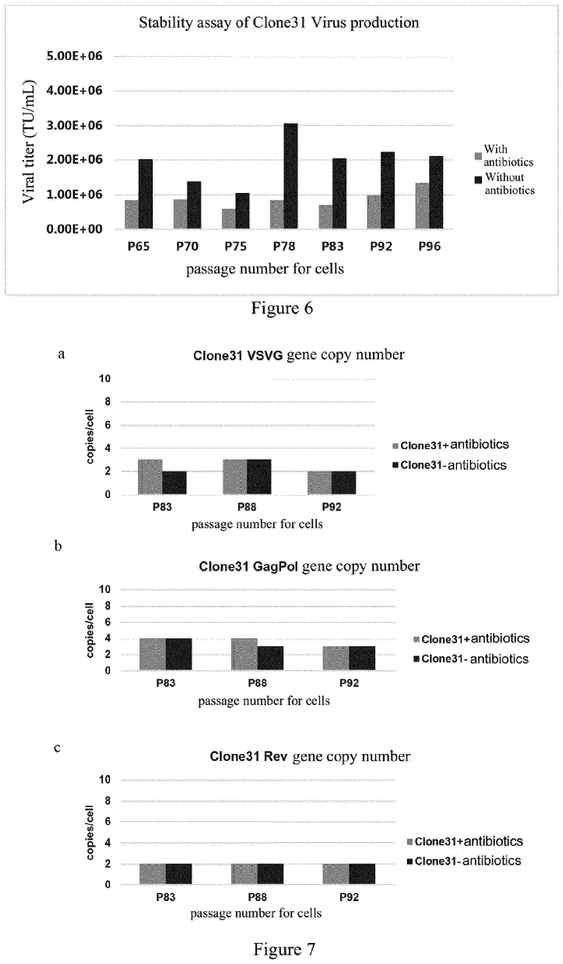

The experimental results showed that when no antibiotics were added, the cells had the ability to produce viruses, indicating that the passaged cells could be inherited normally without the addition of antibiotics (see Fig. 6).

### 7.3 Copy number stability

Clone31 monoclonal cells were validated for copy number stability with and without antibiotics every few generations. The cell genome extraction kit (TIANGEN BIOTECH (BEIJING) CO. LTD., cat. no.: DP304-03) was used to extract the genomic DNA of Clone31 monoclonal cells with and without antibiotics, and then copy numbers of GagPol, Rev and VSVG in the genome of Clone31 monoclonal cells were amplified by the Taqman-qPCR method. In particular, Clone31 cells were inoculated into E125 shake flasks (Corning, Cat: 431145), at a density of 8×10⁵ cells/mL and a volume of 10 mL, respectively. 10 mL of SMM293 TI medium (without antibiotics), and 10 mL of SMM293 TI+0.6 µg/mL Puro (puromycin) + 50 µg/mL Zeo (bleomycin) + 50 µg/mL Hyg (hygromycin) were used, respectively. Cellular genome DNA was extracted every few generations. 2×10⁶ cells/mL were taken for extraction of the genomic DNAs by using the cellular genome extraction kit (TIANGEN BIOTECH (BEIJING) CO., LTD., cat. no.: DP304-03). For operation details, please refer to the manufacturer's instructions for the kit. Cellular genomic DNA was diluted to approximately 5 ng/µL for qPCR. The Taqman-qPCR probe method comprises the following operation steps: diluting three standard plasmid DNAs with a nucleic acid diluent to 1×10⁶ copies/µL, 1×10⁵ copies/µL, 1×10⁴ copies/µL, 1×10³ copies/µL, and 1×10² copies/µL, respectively. The three genes GagPol, Rev, and VSVG were amplified, respectively. Three standard curves were plotted by taking the logarithm of the copy number of the standard product as the abscissa, and the CT value as the ordinate. The qPCR reaction system (Takara, cat. no.: RR390A) comprises: 10 µL of 2×Premix Ex Taq (Probe qPCR), 0.4 µL of F primer (10 µmol/L), 0.4 µL of R primer (10 µmol/L), 0.4 µL of TaqMan Probe (5 µmol/L), 0.4 µL of ROX Reference DyeII (50×), 2 µL of template, 6.4 µL of ddH₂O; wherein, F primers and R primers are listed below:
(1) qPCR primers for VSVG gene:
   VSVG-q-F: 5'-AAGAAACCTGGAGCAAAATCAGA-3'
   VSVG-q-R: 5'-CCTGGGTTTTTAGGAGCAAGATAG-3'
   VSVG-Taqman-Probe: 5'-FAM-CGGGTCTTCCAATCTCTCCAGTGGATCT-TAMRA-3'
(2) qPCR primers for GagPol gene:
   GagPol-q-F: 5'-GCAGTTCATGTAGCCAGTGGATAT-3'
   GagPol-q-R: 5'-TGGTGAAATTGCTGCCATTG-3'
   GagPol-Taqman-Probe:
   5'-FAM-CAGAGACAGGGCAAGAAACAGCATACTTCC-TAMRA-3'
(3) qPCR primers for Rev gene:
   Rev-q-F: 5' -CCTTGGAATGCTAGTTGGAGTAATAA-3'
   Rev-q-R: 5'-TGTTAATTTCTCTGTCCCACTCCAT-3'
   Rev-Taqman-Probe: 5'-FAM-TCTCTGGAACAGATTTGGAATCACACGACC-TAMRA-3'

The qPCR reaction procedure was: 95°C for 30s, 40 cycles of 95°C for 5s, 55°C for 15s, 72°C for 35s. The obtained CT value was placed into the standard curve to calculate the copy number, and then calculate the copy number of each cell based on the mass.

The copy number of VSVG gene for Clone31 is shown in Fig.7a, the copy number of GagPol gene for Clone31 is shown in Fig.7b; and the copy number of Rev gene for Clone31 is shown in Fig. 7c. The experimental results showed that the copy numbers of GagPol, Rev and VSVG genes in the Clone31 monoclonal cells were relatively stable.

### Example 8 Comparison between stable packaging cell line and 293 cells after transfection

The Clone31 monoclonal cells and 293 cells were transfected and compared. 3 batches were made, with 3 parallel groups for each batch. The Clone31 monoclonal cells were transfected and induced under the optimized conditions, and 293 cells were transfected under transient transfection conditions (the PEI amount: plasmid amount = 4:1) for comparison of transfection. Specifically, 293 cells and Clone31 cells were inoculated into E125 shake flasks (Corning, cat. no.: 431145) at a density of 8×10⁵ cells/mL and a volume of 10 mL, respectively. 10 mL SMM293 TI+0.1% F68 medium was used. The cells were placed in three E125 shake flasks, i.e. three groups in parallel. Clone31 monoclonal cells were transfected and induced under optimized conditions. The experimental scheme was: the amount of plasmid being 0.4 [µg/10⁶ cells, the PEI (polyethyleneimine) amount: plasmid amount=4:1, the concentration of sodium butyrate being 10mM, the time for adding sodium butyrate being 24h after transfection, the time for change of sodium butyrate being 6-8 hours after adding sodium butyrate, the time for adding dox (doxycycline) being 24h, the concentration of the added dox (doxycycline) being 2 µg/mL, and the time for collecting the viruses being 48 hours.

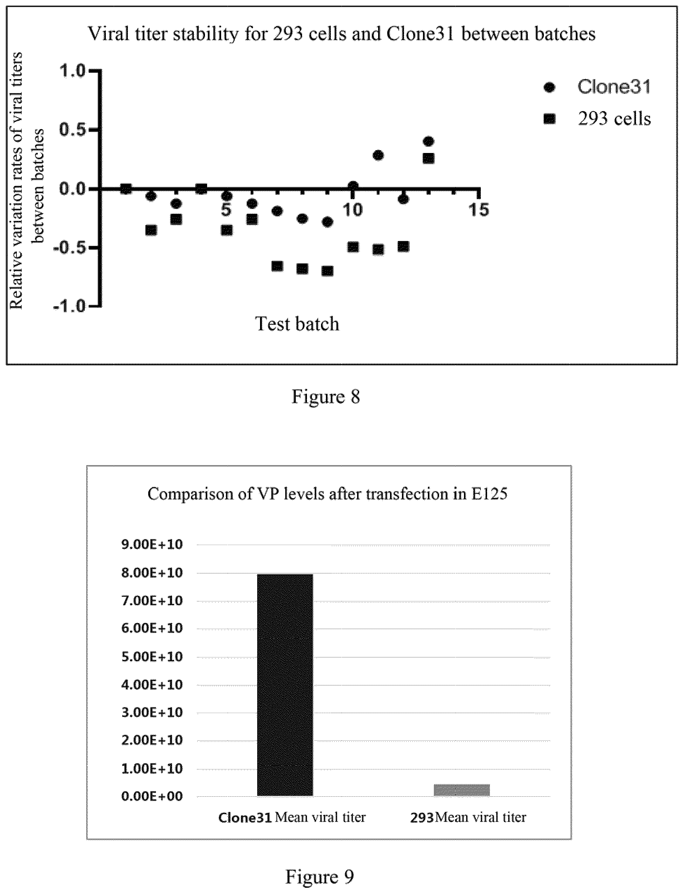

The conditions for transient transfection of 293 cells include: 293 cells were transfected with the pPuro.coTetR plasmid, pVSVG plasmid and pGagPol-RRE-NES-cINT plasmid as constructed in Example 1 and the target plasmids constructed in Example 4 simultaneously. The amount of plasmids for transient transfection within a certain range has little effect on the viral titer, for example, as long as the total amount of plasmids is ranging from 0.8 µg to 1.0 [µg/10⁶ cells. In this example, transfected cells were compared with the mass ratio of the pPuro.coTetR plasmid, pVSVG plasmid, pGagPol-RRE-NES-cINT plasmid and the target plasmid being 1:1:1:2, the total plasmid amount being 0.8 [µg/10⁶ cells (i.e., 0.16 [µg/10⁶ cells of the pPuro.coTetR plasmid, 0.16 [µg/10⁶ cells of the pVSVG plasmid, 0.16 [µg/10⁶ cells of the pGagPol-RRE-NES-cINT plasmid, and 0.32µg/10⁶ cells of the target plasmid), and the PEI (polyethyleneimine) amount: plasmid (Puro.coTetR plasmid + pVSVG plasmid + pGagPol-RRE-NES-cINT plasmid + target plasmid) amount = 4:1. The viral titer stability and viral titer VP (P24) were compared between batches after transfection (see Figs. 8 and 9).

As can be seen from the experimental results in Fig. 8, the Clone31 monoclonal cells had better viral titer stability between batches than 293 cells; as can be seen from the experimental results in Fig. 9, the Clone31 monoclonal cells had much higher viral titer VP (P24) than 293 cells, and Clone31 monoclonal cells had a high content of viral titer VP (P24).

### Example 9 Scale-up process of stable packaging cell line

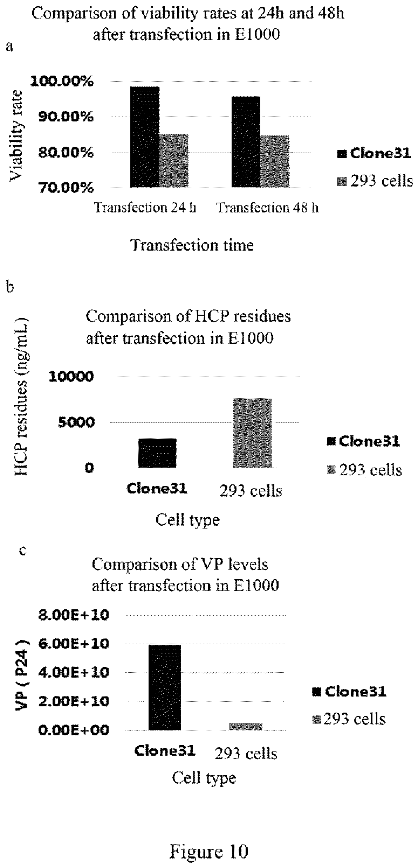

The Clone31 monoclonal cells were scaled up from E125 to E1000 shake flasks (Corning, cat. no.: 431147), and then Clone31 monoclonal cells was transfected with the target plasmids (as constructed in Example 4) for validating virus production. In the meantime, 293 cells were transfected as the control, and the conditions for transfecting the Clone31 monoclonal cells and 293 cells were the same as those described in Example 8. The transfected cells were compared in terms of their cell viability, viral titer VP (Takara, cat. no.: 632200) and HCP (host cell protein) (Cygnus, cat. no.: F650) residues (see Fig. 10).

The experimental results in Fig. 10a indicates that the Clone31 monoclonal cells had higher cell viability than 293 cells at 24h and 48h after transfection; the experimental results in Fig. 10b indicates that the Clone31 monoclonal cells had 1/3 HCP (host cell protein) residues of the 293 cells, i.e., having less HCP (host cell protein) residues than the 293 cells; and the experimental results in Fig. 10c indicates that the Clone31 monoclonal cells had 10-20 times of viral titer VP (P24) than the 293 cells, i.e., having a high content of viral titer VP (P24).

## Claims

1. A stable lentivirus packaging cell line, comprising a packaging plasmid set consisting of a pPuro.coTetR plasmid, a pVSVG plasmid and a pGagPol-RRE-NES-cINT plasmid;
the pPuro.coTetR plasmid comprises a CoTetR gene, the pVSVG plasmid comprises a VSVG gene, and the pGagPol-RRE-NES-cINT plasmid comprises GagPol and Rev genes.

2. The stable lentivirus packaging cell line according to claim 1, wherein, the pPuro.coTetR plasmid further comprises a CMV promoter, a chimeric intron, an SV40 promoter, a puromycin resistance gene, and a polyadenylation signal;
the pVSVG plasmid further comprises a hybrid promoter consisting of a CMV promoter and two tetracycline operators, an SV40 promoter, a bleomycin resistance gene, and a polyadenylation signal; and
the pGagPol-RRE-NES-cINT plasmid further comprises a hybrid promoter consisting of a CMV promoter and two tetracycline operators, a chimeric intron, an SV40 promoter, a hygromycin resistance gene, a cPPT/CTS element, a RRE element, and a polyadenylation signal.

3. The stable lentivirus packaging cell line according to claim 1 or 2, wherein, the CoTetR gene has a sequence as shown in SEQ ID NO: 1; the VSVG gene has a sequence as shown in SEQ ID NO: 2; and the GagPol and Rev genes have a sequence as shown in SEQ ID NO: 3 and 4, respectively.

4. The stable lentivirus packaging cell line according to any one of claims 1-3, wherein, the pPuro.coTetR plasmid has a sequence as shown in SEQ ID NO: 5; the pVSVG plasmid has a sequence as shown in SEQ ID NO: 6; and the pGagPol-RRE-NES-cINT plasmid has a sequence as shown in SEQ ID NO:7.

5. The stable lentivirus packaging cell line according to any one of claims 1-4, wherein the cell line is one selected from the group consisting of HEK293, HEK293-T, HEK293-SF, TE671, HT1080 and HeLa cell lines;
preferably, the cell line is a HEK293 cell line.

6. A method for preparing the stable lentivirus packaging cell line according to any one of claims 1-5, comprising the steps of:
1) linearizing the pPuro.coTetR plasmid, pVSVG plasmid and pGagPol-RRE-NES-cINT plasmid by digestion with an enzyme, respectively;
2) co-transfecting cells with the linearized pPuro.coTetR plasmid and pVSVG plasmid, and adding a first antibiotic for screening to obtain first positive monoclonal cells; and
3) transfecting the first positive monoclonal cells with the linearized pGagPol-RRE-NES-cINT plasmid, and adding a second antibiotic for screening to obtain the second positive monoclonal cells, i.e., stable lentivirus packaging cell lines.

7. The method according to claim 6, wherein, in step 2), polyethyleneimine is further added during the co-transfection of the cells with the linearized pPuro.coTetR plasmid and pVSVG plasmid; and the mass ratio of polyethyleneimine to the plasmids is polyethyleneimine: (pPuro.coTetR plasmid + pVSVG plasmid) = (1-8): 1;
preferably, the mass ratio of polyethyleneimine to the plasmids is: polyethyleneimine: (pPuro.coTetR plasmid + pVSVG plasmid) = (2-5):1;
more preferably, the mass ratio of polyethyleneimine to the plasmids is: polyethyleneimine: (pPuro.coTetR plasmid + pVSVG plasmid) = 4:1; and/or,
in step 3), polyethyleneimine is further added during the transfection of the first positive monoclonal cells with the linearized pGagPol-RRE-NES-cINT plasmid; and the mass ratio of polyethyleneimine to the pGagPol-RRE-NES-cINT plasmid is (1-8): 1;
preferably, the mass ratio of polyethyleneimine to the pGagPol-RRE-NES-cINT plasmid is (2-5):1;
more preferably, the mass ratio of polyethyleneimine to the pGagPol-RRE-NES-cINT plasmid is 4:1.

8. The method according to claim 6 or 7, wherein, in step 2), the pPuro.coTetR plasmid and the pVSVG plasmid are both used in an amount of (0.2-1.0) [µg/10⁶ cells;
preferably, the pPuro.coTetR plasmid and the pVSVG plasmid are both used in an amount of (0.4-0.9) [µg/10⁶ cells;
more preferably, the pPuro.coTetR plasmid and the pVSVG plasmid are both used in an amount of 0.8 [µg/10⁶ cells; and/or,
in step 3), the pGagPol-RRE-NES-cINT plasmid is used in an amount of (0.2-1.0) [µg/10⁶ first positive monoclonal cells;
preferably, the pGagPol-RRE-NES-cINT plasmid is used in an amount of (0.4-0.9) [µg/10⁶ first positive monoclonal cells;
more preferably, the pGagPol-RRE-NES-cINT plasmid is used in an amount of 0.8 [µg/10⁶ first positive monoclonal cells.

9. The method according to any one of claims 6-8, wherein, in step 2), the first antibiotic is a combination of puromycin and bleomycin, which are used in an amount of 1-5 µg/mL and 300-500 µg/mL, respectively; more preferably, puromycin and bleomycin are used in an amount of 2 µg/mL and 400 µg/mL, respectively; and/or,
in step 3), the second antibiotic is a combination of puromycin, bleomycin and hygromycin, wherein, the puromycin, bleomycin and hygromycin are used in an amount of 1-5 µg/mL, 300-500 µg/mL and 300-500 µg/mL, respectively; more preferably, the puromycin, bleomycin and hygromycin are used in an amount of 2 µg/mL, 400 µg/mL and 400 µg/mL, respectively.

10. A method of producing lentiviruses, wherein the stable lentivirus packaging cell line according to any one of claims 1-5 is transfected with a target plasmid.

11. The method according to claim 10, comprising transfecting the stable lentivirus packaging cell line with the target plasmid, and then adding an inducing agent to induce the stable lentivirus packaging cell line to produce lentiviruses.

12. The method according to claim 10 or 11, wherein, polyethyleneimine is further added during the transfection of the stable lentivirus packaging cell line with the target plasmid; and the mass ratio of polyethyleneimine to the target plasmid is polyethyleneimine: the target plasmid = (1-8):1;
preferably, the mass ratio of polyethyleneimine to the target plasmid is polyethyleneimine: the target plasmid = (2-5): 1;
more preferably, the mass ratio of polyethyleneimine to the target plasmid is polyethyleneimine: the target plasmid = 4: 1.

13. The method according to any one of claims 10-12, wherein the target plasmid is used in an amount of (0.2-0.8) µg/10⁶ stable lentivirus packaging cell line;
preferably, the target plasmid is used in an amount of (0.3-0.5) [µg/10⁶ stable lentivirus packaging cell line;
more preferably, the target plasmid is used in an amount of 0.4 [µg/10⁶ stable lentivirus packaging cell line.

14. The method according to any one of claims 10-13, wherein the inducing agent is doxycycline;
preferably, the time for adding the inducing agent is 20-30h after transfection; more preferably, the time for adding the inducing agent is 24h after transfection;
preferably, the inducing agent is added at a concentration of 1-5 µg/mL; more preferably, the inducing agent is added at a concentration of 2 µg/mL.

15. The method according to any one of claims 10-14, wherein an enhancing agent is further added during the production of lentiviruses to increase the lentiviral expression level;
the enhancing agent is sodium butyrate;
preferably, the enhancing agent is added at a concentration of 5-15mM; more preferably, the enhancing agent is added at a concentration of 10mM;
preferably, the time for adding the enhancing agent is 20-30 hours after transfection; more preferably, the time for adding the enhancing agent is 24 hours after transfection;
preferably, the time for changing the enhancing agent is 6-8 hours after adding the enhancing agent; and/or,
the time for collecting the lentivirus is 45-50 hours; preferably, the time for collecting the lentivirus is 48 hours.
